# DEMANDE DE BREVET EUROPEEN

(11) **EP 1 112 748 A2**
(43) Date de publication de la demande: **04.07.2001**
(21) Numéro de dépôt: 01102392.6
(22) Date de dépôt: 21.01.1994
(51) Int. Cl.: A61K 41/00, G01N 33/487, G01N 33/483

(54) **Procédé et dispositif de transmission sous forme de signal de l'activité biologique d'une matière porteuse à une autre matière porteuse, et de traitement d'un tel signal, et produit obtenu avec un tel procédé**

(30) Priorité: 21.01.1993 FR 9300612
(62) Demande divisionnaire de: 94905145.2
(71) Demandeur: BENVENISTE, Jacques, F-75014 Paris (FR)
(72) Inventeur: BENVENISTE, Jacques, F-75014 Paris (FR)
(74) Mandataire: Gutmann, Ernest

(57) **Abrégé**

Il s'agit d'un procédé et dispositif de transmission sous forme d'un signal caractéristique de la manifestation de l'activité biologique ou du comportement biologique spécifique à une substance déterminée, et du traitement d'un tel signal, à partir d'une première matière porteuse présentant ladite activité biologique à une deuxième matière physiquement séparée de la première matière et initialement exempte de toute présence physique de ladite substance déterminée, et d'une matière obtenue par un tel procédé. Ce procédé comporte l'amplification du signal électrique ou électromagnétique émis par la première substance, et capté par un capteur, et la transmission à un émetteur, d'un signal caractéristique de la manifestation de l'activité biologique ou du comportement biologique présentée par la première matière, puis la détection dans la seconde matière d'un signal caractéristique de la manifestation de l'activité biologique spécifique à ladite substance déterminée et transmis à cette deuxième matière par l'intermédiaire des moyens d'amplification à haut gain.

## Description

La présente invention concerne un procédé et un dispositif de transmission sous forme d'un signal de l'activité biologique ou du comportement biologique spécifique à une substance déterminée, à partir d'une première matière porteuse présentant ladite activité biologique, à une deuxième matière porteuse initialement exempte de toute présence physique de ladite substance déterminée. Elle cdncerne également un produit obtenu avec un tel procédé.

Par "activité biologique" on entend toute activité susceptible d'être exercée par une substance biologique à l'égard d'une autre substance plus commodément appelée cible.

La cible peut être simple ou complexe, comme par exemple une molécule, un organe, un être vivant, en particulier lorsque l'activité biologique concernée n'implique pas la réalisation d'une liaison chimique stable entre la substance et la cible.

L'activité biologique spécifique peut être celle d'une substance naturelle ou celle d'une substance artificielle créée par l'homme.

L'expression "substance" telle qu'elle est utilisée ici pour des raisons de commodité de langage, ne doit pas être considérée comme ne s'appliquant qu'à une molécule chimique pure ou individualisée. Elle doit également et notamment être entendue comme englobant tout réactif complexe susceptible de manifester une activité biologique qui serait propre à l'ensemble des éléments dont le réactif pourrait être constitué.

Comme indiqué d'ores et déjà ci-dessus, et en résumé, il résulte donc, de ce qui précède que l'expression "cible" doit quant à elle également être prise dans son sens le plus large, pour être utilisable aussi bien et, selon le cas, (à titre d'exemples) pour une molécule individualisée, par exemple un substrat spécifique d'une enzyme, lorsque celle-ci constitue la "substance" susdite, et pour un organe d'un être vivant lorsque c'est à son égard qu'est testée "l'activité biologique" de la "substance" à l'étude.

L'invention trouve une application particulièrement importante bien que non exclusive dans le domaine de la fabrication de médicaments homéopathiques présentant une activité biologique correspondant à un ou plusieurs principes actifs.

L'invention met à profit une propriété extraordinaire de la matière qui a été mise en lumière par un certain nombre d'expériences dont les résultats sont décrits plus loin, à savoir qu'il est possible de transmettre par des moyens électroniques ou électromagnétiques ne mettant en jeu ou pratiquement en jeu qu'un circuit électronique d'amplification à haut gain, l'expression d'une activité biologique spécifique d'une matière la présentant à une autre matière ne présentant pas initialement ladite activité.

La base physique du procédé selon l'invention est encore inconnue. Elle s'explique peut-être par l'hypothèse suivante : la manifestation de toute activité biologique d'origine moléculaire mettrait en oeuvre à tout le moins partiellement, une activité de type électrique ou électromagnétique. Des observations comme l'inhibition de l'activité biologique par un champ magnétique confortent cette hypothèse, [L.Hadji, B. Arnoux, J.Benveniste (1991) Effect of dilute histamine on coronary flow of guinea-pig isolated heart. Inhibition by a magnetic field, Faseb J. 5:A1583. Voir aussi : J.C. Weaver, R.D. Astumian (1990) The response of living cells to very weak electric fields: the thermal noise limit. Science 247:459462 ; R. Pool (1990) Electromagnetic fields: the biological evidence. Science 249:1096-1098 ; Smith C.W., Best S. (1989) The Electromagnetic Man. J.M. Dent and Sons Ltd., Avon, U.K.].

On connaît également un document (FR 2.634.381) décrivant un procédé pour la fabrication de médicaments homéopathiques en une seule opération quelque soit la dilution choisie.

Aucun exemple de mise en oeuvre du procédé montrant un résultat reproductible ou vérifiable par l'homme du métier n'y est cependant décrit ou indiqué. Ce document fait par ailleurs état de la nécessité de prévoir nécessairement de moyens générateurs hautes fréquences agencés pour superposer un signal alternatif haute fréquence aux signaux qui seraient transmis par l'appareil décrit en référence au procédé.

Une telle disposition est exclue par la présente invention, qui vise par ailleurs à fournir un procédé simple à mettre en oeuvre et de portée plus générale.

Dans ce but l'invention propose notamment un procédé de transmission sous forme d'un signal caractéristique de la manifestation de l'activité biologique ou du comportement biologique spécifique à une substance déterminée, à partir d'une première matière porteuse présentant ladite activité biologique à une seconde matière physiquement séparée de la première matière et initialement exempte de toute présence physique de ladite substance déterminée, ce procédé comportant à la fois l'exposition respectivement de la première matière porteuse, présentant l'activité biologique, à un capteur de signaux électriques ou électromagnétiques, et l'exposition de la seconde matière à un émetteur de signaux électriques ou électromagnétiques, relié au capteur par l'intermédiaire d'un circuit de transmission et d'amplification caractérisé en ce que on amplifie avec un amplificateur à haut gain le signal électrique ou électromagnétique capté par le capteur, sans générer électriquement dans le circuit d'ondes électromagnétiques additionnelles, pendant un temps suffisant pour permettre la transmission à l'émetteur d'un signal caractéristique de la manifestation de l'activité biologique ou du comportement biologique présentée par la première matière, et pour permettre la détection dans la seconde matière d'un signal transmis à celle-ci par l'intermédiaire des moyens d'amplification à haut gain, ce signal étant révélable dans un protocole expérimental identique ou semblable à celui que l'on mettrait en oeuvre pour rendre compte de la présence de ladite substance déterminée dans un milieu qui la contiendrait.

En d'autres termes, la détection de la manifestation caractéristique de l'activité ou du comportement biologique spécifique de la substance déterminée est révélable par l'action que le signal transmis peut exercer sur un substrat (organisme ou réactifs) lors de la mise en oeuvre d'un protocole expérimental identique ou semblable à celui permettant normalement la mise en évidence de la présence dans la première matière porteuse de ladite substance déterminée, grâce à l'action exercée par cette dernière sur le même substrat.

Les moyens de transmission et d'amplification à haut gain comportent un support ou milieu support propre à véhiculer un flux cohérent d'informations à caractères électromagnétiques ou électriques. Ce support comprend par exemple un câble conducteur de l'électricité ou des moyens permettant l'exploitation d'un faisceau lumineux porteur de lumière cohérente.

Par "moyen d' amplification à haut gain" on entend des moyens caractérisés par un coefficient d' amplification d'un signal électrique ou électromagnétique important, notamment supérieur à 1000 et de préférence supérieur à 10.000.

Par exemple la tension est amplifiée de 100 microvolts à 6 Volts et, simultanément l'intensité de 100nA à 150 mA.

Il est important de noter que le circuit entre capteurs et émetteur est agencé pour ne pas modifier la structure du signal transmis, même s'il l'amplifie.

Dans un mode de réalisation avantageux, on filtre les hautes fréquences, par exemple et notamment celles supérieures à 10 kHz pendant la transmission, de sorte qu'aucune onde alternative de haute fréquence, par exemple supérieure à 10 kHz ne transite via le circuit jusqu'à l'émetteur auquel est exposée la seconde matière.

Les signaux transmis sont donc limités aux basses fréquences.

A titre indicatif, on mentionnera qu'avec de tels dispositifs de transmission et d'amplification à haut gain, les temps d'exposition plus haut sont au moins de l'ordre de 10 mn, à de préférence de l'ordre de 15 minutes.

Egalement avantageusement la première et/ou la seconde matière porteuse sont, ou contiennent, des solvants dits "protoniques", c'est-à-dire capables de libérer et/ou de capter des protons, comme par exemple l'eau, l'éthanol ou tout produit présentant un proton labile lié à un atome electro-négatif, de formule du type R - X - H.

Avantageusement, la première matière porteuse et/ou la seconde matière porteuse sont plus spécifiquement de l'eau, ou des produits aqueux. Elle peut (ou elles peuvent) être constituées par toutes matières imprégnables par de l'eau, même si celle-ci n'est présente qu'en faibles proportions. Dans le cas de l'eau, celle-ci est avantageusement constituée par de l'eau distillée, qui a été préalablement chauffée à une température supérieure à de l'ordre de 70° C pendant un temps supérieur à de l'ordre de 20 minutes.

Avantageusement seuls des signaux électromagnétiques sont captés, amplifiés et transmis entre le capteur et l'émetteur.

Dans un mode de réalisation avantageux le procédé selon l'invention est appliqué au traitement des eaux, par exemple à la dépollution des eaux usées ou contaminées biologiquement.

Encore avantageusement la seconde matière est une matière vivante, par exemple un organe non humain.

Avantageusement le signal transmis est stocké de façon intermédiaire (avant d'être transmis à la seconde matière) sur un support de stockage électro-magnétique de type connu en lui même, comme une bande magnétique, ou après traitement par un convertisseur analogique/numérique sur un support de stockage numérique tel qu'un disque optique, une mémoire informatique etc.

Egalement avantageusement le signal transmis est traité par des méthodes de traitement numérique ou analogique connues en elles-même, afin d'être modifié et correspondre ainsi à l'activité biologique d'une substance présentant un principe actif modifié, optimisé, amplifié, purifié ou sans effets secondaires.

On peut ainsi et notamment influencer (augmenter, s'opposer, voir supprimer) une activité biologique déterminée.

Pour optimiser ou modifier de tels signaux en vue d'obtenir un résultat différent de celui obtenu par le signal correspondant au principe actif initial, on procédera par exemple en testant le principe actif modifié grâce à la mise en oeuvre de différents protocoles expérimentaux connus ou aisés à élaborer pour l'homme du métier et qu'il a à sa disposition pour rendre compte de l'activité d'un principe actif déterminé ou amélioré.

Avantageusement le signal transmis correspond à celui émis par plusieurs substances présentes dans la première matière et il est traité par des méthodes de traitement numérique ou analogique connues en elles-même, pour analyser et mesurer une desdites substances parmi les autres, comme par exemple le taux sanguin de glucose ou d'alcool.

Dans un mode de réalisation avantageux, la substance déterminée est présente en dose homéopathique dans la première matière porteuse.

La dose homéopathique utilisée est alors avantageusement optimisée de façon à permettre une manifestation maximum de l'effet biologique recherché, et ce de façon connue en elle même, à partir de nombreux traités écrits et publiés en l'espèce, comme par exemple "l'homéopathie pratique" du docteur C.BINET paru aux éditions d'ANGLES (1979).

Dans un mode de réalisation avantageux la substance déterminée est présente en dose homéopathique dans la première matière porteuse, avec des dilutions supérieures à la limite indiquée par le nombre d'Avogadro.

Dans un mode avantageux de réalisation, la dilution est une dilution de l'ordre de - log4lM (théorique).

Avantageusement la seconde matière est constituée par des granules homéopathiques.

Les granules homéopathiques sont souvent à base de lactose imprégné de molécules d'eau.

L'invention concerne également une matière en un volume fini et porteuse d'une information caractéristique du comportement biologique spécifique à une substance déterminée, mais en l'absence totale de cette substance déterminée dans ladite matière, cette information étant à caractère électrique ou électromagnétique en raison de sa capacité à être transmissible par des moyens électriques ou électromagnétiques, cette manifestation étant normalement révélable par l'action exercée par cette matière en volume fini à l'égard d'un substrat spécifique à la substance déterminée dans un protocole expérimental identique ou semblable à celui que l'on mettrait en oeuvre pour rendre compte de la présence de ladite substance déterminée dans un milieu qui la contiendrait.

Un procédé particulièrement avantageux pour la production d'une telle matière porteuse est un procédé comportant la mise en relation électrique ou électromagnétique de la même matière, cependant non initialement porteuse de la susdite information et exempte de toute présence physique de ladite substance déterminée, avec un milieu contenant cette dernière, par l'intermédiaire de moyens de transmission de signaux électromagnétiques ou électriques comprenant un appareil muni de moyens récepteurs, de moyens amplificateurs à haut gain et des moyens de filtration des hautes fréquences entre les moyens capteurs au-dessus de 10 kHz.

L'invention propose également un dispositif mettant en oeuvre le procédé selon l'invention, comprenant des moyens capteurs de signaux électriques ou électromagnétiques émis par une première matière et caractéristiques d'une manifestation spécifique d'une activité biologique d'une substance déterminée contenue dans cette première matière, des moyens amplificateurs à haut gain desdits signaux et des moyens émetteurs propres à transmettre des signaux également caractéristiques de l'activité biologique à une deuxième matière autrement dépourvu de tout contact avec la première.

Dans des modes particuliers de réalisation, on a de plus recours à l'une et/ou à l'autre des dispositions suivantes :
- les moyens amplificateurs comportent un circuit électronique d'amplification à haut gain, sous forme d'éléments discrets ou du type semi-conducteur ;
- un filtre coupe haut est connecté auxdits moyens capteurs agencé pour filtrer toutes fréquences dans le circuit au-dessus de de l'ordre de 10 kHz ;
- le filtre coupe haut filtre toutes fréquences au-dessus de l'ordre de 5 kHz, avantageusement au-dessus de 1 kHz ou moins ;
- le circuit électronique d'amplification comprend un transistor de sortie monté en émetteur commun ;
- le capteur et l'émetteur comportent des bobines électromagnétiques ;
- l'alimentation de l'appareil se fait par batterie, ce qui permet d'éviter les perturbations possibles du secteur.

Mais une alimentation à partir du réseau alternatif 220 Volts convertie en faible tension continue, par exemple 9 Volts, est également tout à fait utilisable ;
- le dispositif comprend de plus des moyens support de stockage électro-magnétique des signaux transmis, de type connu en eux même, comme par exemple une bande magnétique;
- le dispositif comporte de plus des moyens convertisseurs analogique/numérique des signaux transmis et des moyens de stockage sur un support de stockage de données numériques desdits signaux, comme par exemple un disque optique, une mémoire informatique etc ;
- le dispositif comprend des moyens de traitement numérique ou analogique du signal transmis, pour modifier le dit signal pour le faire correspondre à celui d'une substance présentant un principe actif modifié, optimisé, amplifié, purifié ou sans effets secondaires ;
- le dispositif comprend des moyens de traitement numérique ou analogique agencés pour analyser et mesurer les signaux transmis correspondant à une substance parmi d'autres, comme par exemple le taux de glucose dans le sang ou le taux d'alcool ;
- la seconde bobine est agencée pour émettre vers une matière vivante, tel qu'un organe non humain.

La présente invention sera mieux comprise à la lecture de la description qui suit d'un mode de réalisation donné à titre d'exemple non limitatif, et au vu des exemples et résultats fournis ci-après de façon non limitative.

La description se réfère également aux dessins qui l'accompagnent, dans lesquels :
- La figure 1 est un schéma du dispositif selon un mode de réalisation de l'invention.
- La figure 2 est un schéma électrique du dispositif de transmission de la figure 1.

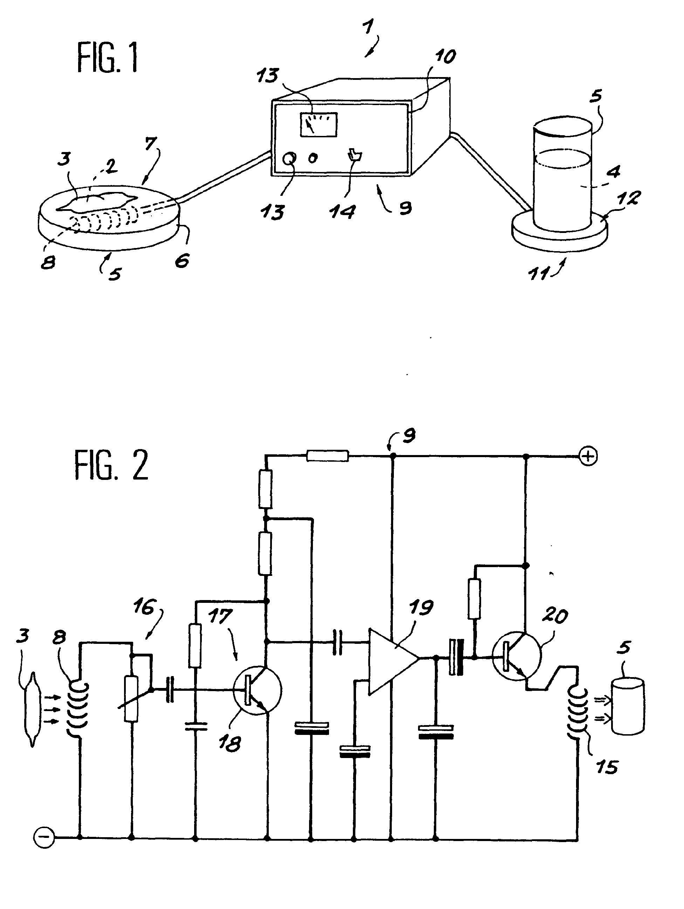

La figure 1 montre un dispositif 1 de transmission de l'activité biologique spécifique à une substance déterminée, par exemple de l'histamine ou de l'ovalbumine, d'une première matière porteuse 2, par exemple constituée par de l'eau distillée placée dans une ampoule de préférence en verre 3 de 1 à 10 ml, à une deuxième matière 4 également constituée par de l'eau distillée et placée dans une ampoule de 1 à 10 ml ou un récipient 5 par exemple de 500 ml, voire plus, également et de préférence en verre.

La matière 2 peut comporter en son sein la présence physique de la substance déterminée, en quantité ou non homéopathique, ou peut simplement comporter les informations caractéristiques de l'activité ou du comportement biologique spécifique à la substance déterminée.

La présence physique peut par exemple être révélée par une méthode du type spectrométrie ou spectrofluorométrie.

Les informations caractéristiques d'une manifestation elle-même caractéristique du comportement biologique spécifique à une substance déterminée peut, quand à elle, être normalement révélable à l'occasion de l'action qu'elle peut exercer sur un milieu contenant cette substance déterminée à l'égard d'une cible (organisme ou réactifs) mis en oeuvre dans un protocole expérimental apte à rendre compte de la présence dans ce milieu de ladite substance déterminée.

La matière 4 est initialement exempte en son sein de toute présence physique de ladite substance déterminée, et n'est pas initialement porteuse d'informations caractéristiques du comportement biologique spécifique à la substance déterminée.

Le dispositif 1 comprend un capteur électromagnétique 5 comportant un boitier 6, muni d'un plateau 7 sur lequel l'ampoule 3 est déposée.

Dans le mode de réalisation plus particulièrement décrit ici, le plateau est par exemple constitué en matière plastique transparente aux ondes électromagnétiques, de faible épaisseur (par exemple 2 millimètres).

A l'intérieur du boîtier 6 se trouve le capteur électromagnétique proprement dit, par exemple constitué par une bobine réceptrice 8 comme on le verra en référence à la figure 2.

Le capteur 5 est relié, par câble conducteur électrique, à un circuit 9 amplificateur à haut gain placé dans un boîtier 10.

La sortie du circuit 9 est connectée, également par câble conducteur électrique, à un capteur émetteur 11, de configuration similaire au capteur 5 mais agencé pour l'émission, et sur le plateau 12 duquel est placé l'ampoule ou le récipient 5 de rétention de la deuxième matière 4.

Le circuit 9 comprend des moyens 13 de réglage de la puissance (potentiomètre, cadran, etc) et de mise sous tension 14 (interrupteur) du dispositif connus en eux-mêmes.

On a représenté plus précisément sur la figure 2 le circuit électronique 9 du dispositif 1 selon le mode de réalisation de l'invention plus particulièrement décrit ici.

Le circuit 9 est connecté d'un côté à la bobine électromagnétique 8 à haute impédance (réceptrice) (par exemple une bobine constituée d'environ 600 spires de fil émaillé de 5/100 ) appartenant au capteur 5, et de l'autre côté à la bobine électromagnétique 15 à haute impédance (émettrice) (par exemple une bobine constituée de 100 spires de fil émaillé de 20/100) appartenant au capteur 11.

Le circuit 9 comprend un filtre coupe haut 16 (par exemple de 10kHz) connecté à la bobine 8 et un préamplificateur 17 comportant un transistor amplificateur 18.

Le préamplificateur 17 est connecté en sortie à l'amplificateur opérationnel 19 qui peut être connecté directement à la bobine émettrice 15, ou comme représenté sur la figure 2, via un transistor de sortie 20 monté en émetteur commun pour engendrer un courant de sortie de plus forte intensité.

Une telle modification permet de traiter des volumes de liquides plus importants dans le même temps, la tension alternative de sortie étant par ailleurs et par exemple de 4 à 5 Volts crête à crête.

Sinon la disposition ci-dessus garantit à la sortie un signal équivalent à une tension de 3 ou 4 V et un courant d'au moins 20 mA.

Dans une variante avantageuse l'amplificateur est à gain variable par exemple de < 1mV à > 3/4 V et de <10 microampères à > 20 mA.

L'alimentation du circuit permettant la polarisation des transistors se fait avantageusement exclusivement par des batteries (non représentées), ce qui permet d'éviter les modifications incontrôlées de la structure du signal dues à des perturbations imprévisibles du réseau d'alimentation 50 Hz (secteur), en cas d'utilisation d'un transformateur.

On va maintenant faire référence, à titre illustratif, à deux exemples spécifiques de transfert.

Dans le premier cas (exemple n°1), il s'agissait d'un transfert à partir d'eau distillée, des principes actifs de l'ovalbumine (Ova) ou de l'endotoxine de E.Coli (Endo) vers une seconde matière également constituée d'eau distillée, et dans le deuxième cas (exemple n°2) d'un transfert toujours entre deux matières constituées d'eau distillée, des principes de l'endotoxine de E.Coli (Endo) ou de l'histamine (Hista).

Un tableau récapitulatif de plusieurs expériences effectuées par l'inventeur à ce jour, qui ont ainsi pu valider le procédé et le dispositif de l'invention, est également présenté.

La méthode de détection de l'activité biologique utilisée dans les expériences effectuées, dont celles correspondant aux deux exemples ci-après, est la suivante.

Des coeurs de cobayes mâles d'Hartley d'environ 400 g sont montés sur un appareil connus sous la dénomination ANDERSON pour perfusion de coeur et perfusés à 37°C avec une solution tampon Krebs-Henseleit (KHB en initiales anglosaxonnes pour Krebs-Henseleit Buffer) (1mM Ca²⁺) avec un pH de l'ordre de 7,4. La solution est aérée en permanence avec un mélange O₂/CO₂ à 95,5%.

Le débit coronaire est contrôlé en permanence par exemple à l'aide d'un dispositif de pesage automatique connu en lui-même, connecté à des moyens informatiques de traitement et de restitution des débits mesurés, sous forme graphique.

Les contractions systoliques maximales et minimales, le rythme cardiaque et les valeurs de dp/dt (vitesse de la contraction musculaire) sont mesurées et enregistrées en permanence via un transducteur, par exemple un transducteur connu sous la référence ELI-S045-35 de la société **EMKA Technologies** : 53 bld du Général Martial Vallin - 75015 Paris (France).

Les principes actifs (histamine, ovalbumine ou endotoxine d'E.Coli) qui ont fait l'objet d'un transfert, ont été dilués à partir de concentration d'1mM avec de l'eau distillée.

Entre chaque dilution, les solutions étaient violemment agitées dans un vortex pendant 15 s.

Les solutions sont injectées à la base de l'aorte avec une seringue électrique (6 ml ; 1 ml/mn).

### EXEMPLE N°1 :

Le transfert d'ovalbumine (Ova), d'endotoxine de E.Coli (Endo) et, à titre de contrôle, d'eau distillée, en ampoules scellées de 2 ml, a été effectué vers des ampoules-filles scellées d'eau distillée de 2 ml.

La concentration en principe actif était dans les ampoules "émettrices" de 1 x 10⁻⁸ Moles par litre.

Les ampoules-filles ont ensuite été diluées au 1/1000 et 20 ml des dilutions ont été répartis en tubes de 50 ml.

Les tubes ont été testés à l'aveugle (dans l'ordre, de 1 à 12) les 11 et 12 juillet 1992 sur deux coeurs isolés de cobayes préimmunisés à l'ovalbumine.

Les résultats sont les suivants :

| Tubes N° | Principe actif transféré (ampoule-mère) | % variation du flux coronaire | | Principe actif détecté dans le tube récepteur (ampoule-fille) |
|---|---|---|---|---|
| | | Coeur A | Coeur B | |
| 1 | Endo | 50 | 17 | + |
| 2 | Endo | 55 | 21 | + |
| 3 | Ova | 75 | 93 | + |
| 4 | H2OTr* | 0 | 0 | - |
| 5 | Ova | -50 | -53 | + |
| 6 | H2O** | 0 | 0 | - |
| 7 | H2OTr | 0 | 0 | - |
| 8 | H2O | 0 | 0 | - |
| 9 | H2O | 0 | 0 | - |
| 10 | H2OTr | 0 | 0 | - |
| 11 | H20 | 11 | 10 | - *** |
| 12 | Ova | -37 | -42 | + |

| | | | | |
|---|---|---|---|---|
| ^{*} eau ayant reçu une information eau | | | | |
| ^{**} eau d'origine | | | | |
| ^{***} résultat légèrement variant mais néanmoins acceptable. Il s'explique probablement par une contamination bactérienne du tube, donnant une réaction de type endotoxine. | | | | |

Les effets des 5 tubes actifs (eau ayant reçu une information Ova ou Endo) et l'absence d'effet des 7 contrôles (eau d'origine ou ayant reçu une information eau) sont nets et reproductibles.

On retrouve ces différences sur les effets mécaniques (non montrés ici).

Cette expérience illustre la transmission d'activités biologiques à de l'eau par un circuit électronique ou électromagnétique selon l'invention.

### EXEMPLE N°2 :

Les tubes ont été testés le 23 septembre 1992. Les conditions opératoires sont identiques à celles de l'exemple 1.

Les résultats sont les suivants :

| Tubes N° | Principe actif transféré (ampoule-mère) | % variation du flux coronaire | Principe actif détecté dans le tube récepteur (ampoule-fille) |
|---|---|---|---|
| 1 | H₂OTR | 0 | - |
| 2 | H₂OTR chauffée | 0 | - |
| 3 | HistaTR | -10 | + |
| 4 | OvaTR | -94 | + |

### TABLEAU RECAPITULATIF

Le tableau suivant donne les effets sur le flux coronaire en % de variation du débit (en + ou en -) sur des coeurs de cobayes préalablement immunisés à l'ovalbumine (en présence d'Alum comme adjuvant) fin octobre 1992.

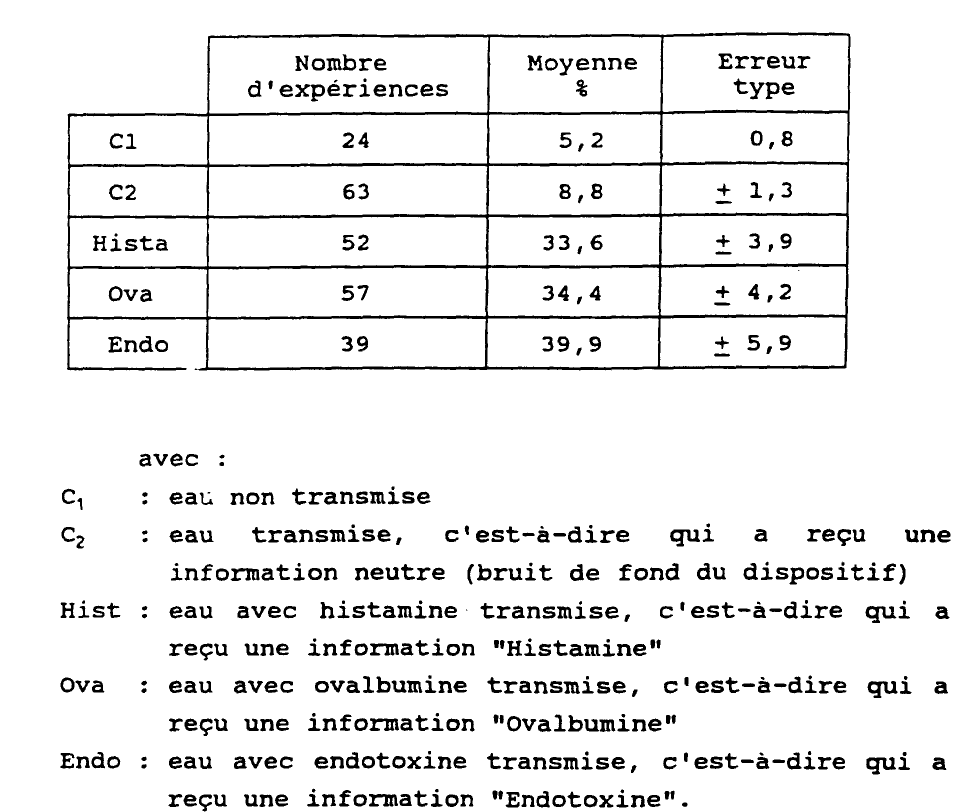

Comme on peut le constater les flux coronaires varient de façon significative et systématique lors du transfert d'informations correspondant à l'histamine, l'ovalbumine ou l'endotoxine, alors qu'en présence d'eau (transmise ou non), de faibles variations ou sensiblement aucune variation sont observées, ce qui illustre la présente invention.

L'utilisation de seconde matière dans laquelle se manifeste l'activité transmise peut se faire par exemple par voie orale, par injection, par imprégnation voire même par contact entre la peau de l'individu à traiter et un récipient contenant ladite seconde matière.

Comme il va de soi, et comme il résulte d'ailleurs de ce qui précède, la présente invention n'est pas limitée aux modes de réalisation de l'invention plus particulièrement décrit. Elle en concerne au contraire toutes les variantes et notamment celles où :
- les matières porteuses ne sont pas de l'eau pure, mais des mélanges aqueux, ou des matières pâteuses ou solides,
- les capteurs ne sont pas de type électromagnétique mais du type électrique, c'est-à-dire qu'ils sont agencés pour détecter une différence de potentiel. Il peut alors s'agir de plaques métalliques reliées entre elles via un amplificateur à haut gain, les première et deuxième matières ou leurs récipients étant notamment placées à contact avec les capteurs,
- les principes actifs sont différents de ceux plus particulièrement testés. Tous types de molécules biologiques susceptibles d'être contenues dans tous types de substances naturelles ou artificielles agissant sur les êtres vivants, espèces animale ou végétale, sont en fait concernés. Il peut s'agir par exemple des substances présentes dans le sang ou tout autre liquide organe ou tissu biologique des animaux ou des hommes in vitro, ex vivo, in vivo.

La révélation de la présence d'informations correspondant à un principe actif pourra alors se faire différemment, de façon connue en elle-même par un homme du métier pour le principe actif concerné.

## Revendications

1. Procédé pour modifier l'activité biologique d'une substance ci-après dite matière réceptrice, ledit procédé étant caractérisé en ce qu'il comporte :
- l'étape d'exposer à un capteur (5) de signaux magnétiques ou électromagnétiques une matière ayant une activité biologique, ci-après dite matière émettrice ; ledit capteur comportant une bobine électromagnétique (8) à haute impédance
ladite activité biologique, due à la présence dans la matière émettrice d'une quantité significative d'une substance active, pouvant être mise en évidence au moyen d'un protocole expérimental ;
ledit procédé étant caractérisé en ce qu'il comporte en outre les étapes suivantes :
- l'étape de filtrer par un filtre coupe-haut au dessus de 10 kHz, les signaux électriques en sortie dudit capteur (5),
- l'étape d'amplifier (9) les signaux électriques ainsi filtrés,
- l'étape d'appliquer les signaux électriques ainsi amplifiés aux bornes d'un émetteur électromagnétique (11) émettant un champ électromagnétique vers la matière réceptrice ; ledit émetteur comportant une bobine électromagnétique (15) ;

2. Procédé selon la revendication 1 ; ledit procédé étant tel que la matière émettrice et/ou la matière réceptrice contiennent de l'eau ou consistent en un produit aqueux.

3. Procédé selon la revendication 2 ; ledit procédé étant tel que l'eau de la matière émettrice et/ou de la matière réceptrice est constituée par de l'eau distillée qui a été préalablement chauffée à une température supérieure un seuil de température de l'ordre de 70 °C et pendant un temps supérieur à un seuil ayant une durée de l'ordre de 20 minutes.

4. Procédé selon l'une quelconque des revendications précédentes ; ledit procédé étant tel que la matière émettrice comporte plusieurs substances actives.

5. Procédé selon l'une quelconque des revendications précédentes ; ledit procédé comprenant en outre :
- l'étape de traiter lesdits signaux électriques par des méthodes de traitement numérique ou analogique.

6. Procédé selon les revendications 4 et 5 ; l'étape de traiter lesdits signaux électriques par des méthodes de traitement numérique ou analogique comprenant en outre :
- l'étape d'analyser ou de mesurer une desdites substances actives parmi les autres.

7. Procédé selon l'une quelconque des revendications précédentes ; ledit procédé comprenant :
- l'étape de stocker lesdits signaux électriques de façon intermédiaire (avant d'être appliqués à la matière réceptrice), notamment sur un support de stockage numérique, notamment après traitement par des méthodes de traitement numérique ou analogique.

8. Procédé selon l'une quelconque dès revendications précédentes, ledit procédé étant tel que la substance active est diluée dans la matière émettrice, avec des dilutions supérieures à la limite indiquée par le nombre d'Avogadro.

9. Procédé selon l'une quelconque des revendications précédentes ; ledit procédé étant tel que la substance active est diluée de manière homéopathique dans la matière émettrice.

10. Procédé selon l'une quelconque des revendications précédentes, ledit procédé étant tel que la matière réceptrice est constituée par des granules homéopathiques.

11. Substance porteuse manifestant une activité biologique spécifique d'une substance active ; ladite activité biologique pouvant être révélée par l'action exercée par ladite substance porteuse dans un protocole expérimental identique ou semblable que l'on mettrait en oeuvre pour rendre compte de la présence de ladite substance active dans un milieu qui la contiendrait ;
ladite substance porteuse ayant été obtenue :
- en exposant à un capteur (5) de signaux magnétiques ou électromagnétiques une composition contenant une quantité significative de ladite substance active,
- en amplifiant (9) les signaux électriques ainsi produits à la sortie dudit capteur (5),
- en appliquant lesdits signaux électriques ainsi amplifiés aux bornes d'un émetteur électromagnétique (15) émettant un champ électromagnétique vers la substance porteuse.

12. Substance porteuse selon la revendication 11, ladite substance étant telle que la substance porteuse et/ou la composition contenant la substance active contiennent de l'eau ou consistent en un produit aqueux.

13. Substance porteuse selon la revendication 12, ladite substance étant telle que l'eau de la substance porteuse et/ou de la composition contenant la substance active est constituée par de l'eau distillée qui a été préalablement chauffée à une température supérieure à une seuil de l'ordre de 70 °C pendant un temps supérieur à un seuil de l'ordre de 20 minutes.

14. Substance porteuse selon l'une quelconque des revendications 11 à 13, ladite substance étant telle que la composition comporte plusieurs substances actives.

15. Substance porteuse selon l'une quelconque des revendications 11 à 14 ; ladite substance porteuse ayant été obtenue :
- en traitant lesdits signaux électriques par des méthodes de traitement numérique ou analogique.

16. Substance porteuse selon la revendication 15; ladite substance porteuse ayant été obtenue :
- en stockant lesdits signaux électriques de façon intermédiaire (avant d'être appliqués à la substance porteuse), notamment sur un support de stockage numérique après traitement par des méthodes de traitement numérique ou analogique.

17. Substance porteuse selon l'une quelconque des revendications 11 à 16 ; ladite substance porteuse étant telle que la substance active est diluée dans la composition, avec des dilutions supérieures à la limite indiquée par le nombre d'Avogadro.

18. Substance porteuse selon l'une quelconque des revendications 11 à 17 ; ladite substance porteuse étant telle que la substance active est diluée de manière homéopathique dans la composition.

19. Substance porteuse selon l'une quelconque des revendications 11 à 18 ; ladite substance porteuse étant constituée par des granules homéopathiques.

20. Utilisation de la matière porteuse selon l'une quelconque des revendications 11 à 19 pour la production d'un agent à caractère thérapeutique dont la destination est liée au comportement biologique spécifique de ladite substance active.

21. Système pour modifier l'activité biologique d'une substance ci-après dite matière réceptrice ;
les applications dudit système au traitement thérapeutique in vivo d'un homme ou d'un animal sont exclues du champ de la présente revendication ;
les applications dudit système à la réalisation d'un agent susceptible d'avoir des effets thérapeutiques entrent dans le champ de la présente revendication ;
ledit système comportant :
- un premier récipient (3) contenant une matière ayant une activité biologique, ci-après dite matière émettrice ;
ladite activité biologique, due à la présence dans la matière émettrice d'une quantité significative d'une substance active, pouvant être mise en évidence au moyen d'un protocole expérimental ;
ledit système comportant en outre :
- un capteur (5) de signaux magnétiques ou électromagnétiques situé à proximité dudit premier récipient (3) ; ledit capteur produisant des signaux électriques ;
ledit système comportant en outre :
- des moyens d'amplification (9) à haut gain pour amplifier les signaux électriques sortant dudit capteur (5),
- un deuxième récipient contenant la matière réceptrice,
- un émetteur électromagnétique (11), situé à proximité dudit deuxième récipient, connecté aux moyens d'amplification (9), recevant les signaux électriques ainsi amplifiés et émettant, vers la matière réceptrice, un champ électromagnétique ;

22. Système selon la revendication 21; ledit système étant tel que la matière émettrice et/ou la matière réceptrice contiennent de l'eau ou consistent en un produit aqueux.

23. Système selon la revendication 22 ; ledit système étant tel que l'eau de la matière émettrice et/ou de la matière réceptrice est constituée par de l'eau distillée qui a été préalablement chauffée à une température supérieure à un seuil de l'ordre de 70 °C pendant un temps supérieur à un seuil de l'ordre de 20 minutes.

24. Système selon l'une quelconque des revendications 21 à 23 ; ledit système étant tel que la matière émettrice comporte plusieurs substances actives.

25. Système selon l'une quelconque des revendications 21 à 24 ; ledit système comprenant en outre :
- des moyens de traitement numérique ou analogique pour traiter lesdits signaux électriques, notamment des moyens de conversion analogique/numérique.

26. Système selon les revendications 24 et 25 ; ledit système étant tel que les moyens de traitement numérique ou analogique comprennent en outre :
- des moyens d'analyse et de mesure pour analyser ou pour mesurer une desdites substances actives parmi les autres.

27. Système selon l'une quelconque des revendications 21 à 26; ledit système comprenant en outre :
- des moyens de stockage pour stocker lesdits signaux électriques de façon intermédiaire (avant d'être appliqués à la matière réceptrice), notamment sur un support de stockage numérique, notamment après traitement par des moyens de traitement numérique ou analogique.

28. Système selon l'une quelconque des revendications 21 à 27, ledit système étant tel que la substance active est diluée dans la matière émettrice, avec des dilutions supérieures à la limite indiquée par le nombre d'Avogadro.

29. Système selon l'une quelconque des revendications 21 à 28 ; ledit système étant tel que la substance active est diluée de manière homéopathique dans la matière émettrice.

30. Système selon l'une quelconque des revendications 21 à 29, ledit système étant tel que la matière réceptrice est constituée par des granules homéopathiques.

31. Système selon l'une quelconque des revendications 21 à 30 ; ledit système comportant en outre :
- un filtre coupe-haut connecté audit capteur et agencé pour filtrer toutes fréquences au-dessus de 10 kHz à la sortie dudit capteur.

32. Système selon l'une quelconque des revendications 21 à 31, ledit système étant tel que le capteur (5) et l'émetteur (11) comportent des bobines électromagnétiques (8, 15).
